# EUROPEAN PATENT APPLICATION

(11) **EP 1 574 499 A1**
(43) Date of publication of application: **14.09.2005**
(21) Application number: 04005498.3
(22) Date of filing: 08.03.2004
(51) Int. Cl.: C07D 209/48, C07D 233/66, C07D 471/04, A61K 31/4035, A61P 35/00

(54) **Inhibitors of DNA methylation in tumor cells**

(71) Applicant: DKFZ Deutsches Krebsforschungszentrum, 69120 Heidelberg (DE)
(72) Inventor: Garcia Boy, Regine, 52319 Iowa (US); Lyko, Frank, 69115 Heidelberg (DE); Siedlecki, Pawel, 00-560 Warschau (PL)
(74) Representative: Huhn, Michael

(57) **Abstract**

The present invention relates to compounds according to the general formula 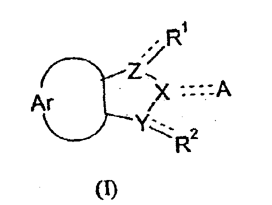 wherein the dotted lines denote a single bond which is optionally present, with 2 dotted lines denoting a double bond; and
wherein the symbols in particular have the following meanings:
R¹ and R² are independently from each other selected from the group consisting of:
H; OH; (=O); halogens; pseudohalogens; NH₂; S(O)ₘR⁵; SO₂NH₂; C(O)R⁸; C(O)OR⁹; CONH₂; C₁-C₂-alkyl substituted by NH₂, OH, S(O)ₘR⁵, SO₂NH₂, C(O)R⁸, C(O)OR⁹, CONH₂; C₁-C₂-alkoxy substituted by NH₂, OH, S(O)ₘR⁵, SO₂NH₂, C(O)R⁸, C(O)OR⁹, CONH₂;
Ar denotes an unsubstituted mononuclear aryl group having 6 or 7 members, which aryl group is annulated to the neighbouring 5-membered cycle, and which may carry 1, 2 or 3 heteroatoms from the group N, O and S in its cycle;
Y, Z denote independently from each other a nitrogen atom or a methylene group;
X is a nitrogen atom or a methylene group;
A is selected from the group consisting of: H; halogens and pseudohalogens; OH; =N(OH); NR¹²R¹³; OSO₃ ⁻; S(O)ₘR¹⁴; SO₂NR¹⁵R¹⁶; C(O)R¹⁷; C(O)OR¹⁸; CONR¹⁹R²⁰; C(S)R²¹, C(S)OR²²; unsubstituted and at least monosubstituted C₁-C₆-alkyl which can carry in its chain one or more non-adjacent heteroatoms from the group nitrogen and oxygen, and which, if substituted, carry at least one substituent which is preferably selected from the group consisting of: halogens, pseudohalogens, OH, NR¹²R¹³, OSO₃⁻, S(O)ₘR¹⁴, SO₂NR¹⁵R¹⁶, C(O)R¹⁷, C(O)OR¹⁸, CONR¹⁹R²⁰, C(S)R²¹, C(S)OR²², and substituted and non-substituted aryl and substituted and non-substituted heteroaryl which, if substituted, carry at least one substituent from the group consisting of C₁-C₃-alkyl, C₁-C₃-alkoxy, halogens, pseudohalogens, and CF₃.

These compounds lend themselves for the manufacture of drugs. They are useful in the inhibition of DNA methylation, the inhibition of DNA methyltransferases, and may therefore be useful for the manufacture of pharmaceuticals for the treatment of developmental disorders such as Prader-Willi-Syndrome, Angelman-Syndrome (Happy Puppet Syndrome), Beckwith-Wiedemann-Syndrome, and proliferative diseases, such as coronary restenosis and neoplastic diseases, particularly colon carcinoma, familiary adenomatous polyposis carcinoma and hereditary non-polyposis colorectal cancer, prostate carcinoma, melanoma, non-Hodgkin lymphoma, acute lymphatic leukemia (ALL), chronic lymphatic leukemia (CLL), acute myeolid leukemia (AML), chronic myeloid leukemia (CML), or hepatocellular carcinoma. These compounds may also be used for other applications including the induction of cellular differentiation, diagnosis, and the use in screening assays.

## Description

The present invention relates to compounds of the general formula (I), with the definitions of R¹ to R² given below in the text, and the use of these compounds and/or pharmaceutically acceptable salts thereof as a pharmaceutical. The compounds according to formula (I) lend themselves in particular as inhibitors of DNA methylation in cells, particularly tumor cells.

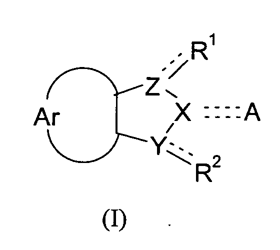

DNA can be methylated through covalent methylation of cytosine residues at their carbon-5 position. It has been found that DNA methylation is an important mechanism of gene regulation, particularly gene silencing. Gene regulation by DNA methylation is an "epigenetic" form of gene regulation, as the DNA sequence information itself remains unaltered.

Aberrant DNA methylation patterns are closely associated with epigenetic mutations or epimutations, which can have the same consequences as genetic mutations. For example, many tumors show hypermethylation and concomitant silencing of tumor suppressor genes. Several developmental disorders are also associated with aberrant DNA methylation.

Thus, changes in DNA methylation play an important role in developmental and proliferative diseases, particularly in tumorigenesis.

The DNA methylation reaction is catalyzed by DNA methyl transferases (DNMTs). Establishment and maintenance of DNA methylation patterns require the activity of several DNMTs. In mammalians, DNA methylation is established during early embryogenesis by the de novo DNA methyl transferases (DNMT3A and DNMT3B). In differentiated cells, methylation patterns are maintained by DNMT1 which is therefore also responsible for maintenance of epimutations.

Inhibition of DNA methylation, particularly by inhibition of DNMTs, more particularly DNMT1, is considered a promising strategy for treatment of proliferative diseases.

However, satisfying methods for inhibition of DNA methylation have previously not been available.

Genetic inhibition of DNMTs, although theoretically possible (US 6,054,439), suffers from the widely known and currently insurmountable difficulties associated with gene therapy.

Pharmacological inhibition of DNMTs has been limited to the use of structural analogues of cytosine, such as 5-azacytidine, 5-aza 2'deoxycytidine (decitabine), and 5,6-dihydro-5-azacytidine (US 4,058,602; DE 198 23 484 A1). These cytosine analogues suffer from low specificity and high toxicity, limiting their use to a very small set of clinical indications.

Therefore, there is a need for other inhibitors of DNA methylation, particularly inhibitors of DNMTs. Preferably, such inhibitors should have a different mode of action than structural analogues of cytidine, and they should be more specific and less toxic than other inhibitors of DNA methylation. The object of the present invention is to provide compounds and pharmaceuticals showing said abilities.

This object is attained by compounds according to the general formula 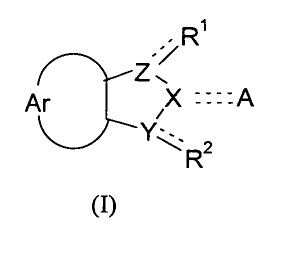 wherein the dotted lines denote a single bond which is optionally present, with 1 dotted line and 1 full line or 2 dotted lines denoting a double bond; and
wherein the symbols have the following meanings:
R¹ and R² are independently from each other selected from the group consisting of:
   H; (=O); OH; OSO₃⁻; halogens; pseudohalogens; NR³R⁴; S(O)ₘR⁵; SO₂NR⁶R⁷; C(O)R⁸; C(O)OR⁹; CONR¹⁰R¹¹; substituted and unsubstituted C₁-C₃-alkyl and substituted and unsubstituted C₁-C₃-alkoxy, which alkyl and alkoxy groups, if substituted, carry at least one substituent from the group: OH, OSO₃⁻, halogens, pseudohalogens, NR³R⁴, S(O)ₘR⁵, SO₂NR⁶R⁷, C(O)R⁸, C(O)OR⁹, CONR¹⁰R¹¹, and wherein at least one substituent is different from H;
   Ar denotes a substituted or unsubstituted mononuclear aryl group having 6 or 7 members, which aryl group is annulated to the neighbouring 5-membered cycle, and which may carry 1, 2 or 3 heteroatoms from the group N, O and S in its cycle;
   Y, Z denote independently from each other a nitrogen atom, an oxygen atom, a sulfur atom or a methylene group;
   X is a nitrogen atom, an oxygen atom, a sulfur atom, or a methylene group;
   A is selected from the group consisting of: H; halogens and pseudohalogens; OH; =N(OH); NR¹²R¹³; OSO₃⁻; S(O)ₘR¹⁴; SO₂NR¹⁵R¹⁶; C(O)R¹⁷; C(O)OR¹⁸; CONR¹⁹R²⁰; C(S)R¹¹, C(S)OR²²; unsubstituted and at least monosubstituted C₁-C₁₂-alkyl which can carry in its chain one or more non-adjacent heteroatoms from the group nitrogen and oxygen, and which, if substituted, carry at least one substituent which is preferably selected from the group consisting of: halogens, pseudohalogens, OH, NR¹²R¹³, OSO₃-, S(O)ₘR¹⁴, SO₂NR¹⁵R¹⁶, C(O)R¹⁷, C(O)OR¹⁸, CONR¹⁹R²⁰, C(S)R²¹, C(S)OR²², and substituted and non-substituted aryl and substituted and non-substituted heteroaryl which, if substituted, carry at least one substituent from the group consisting of C₁-C₃-alkyl, C₁-C₃-alkoxy, halogens, pseudohalogens, and CF₃;
R³, R⁴ are independently selected from the group consisting of:
   H; substituted and unsubstituted methyl and ethyl which, if substituted, can carry one or more substituents from the group OH, halogens, pseudohalogens,;
R⁵ independently has the same meaning as R³;
R⁶ and R⁷ independently have the same meaning as R³, R⁴;
R⁸ is H or C₁-C₃-alkyl which can be unsubstituted or carry one or more substituents from the group consisting of OH, C(O)H, C(O)CH₃, C(O)C₂H₅, halogens, pseudohalogens, NH₂, mono(C₁-C₃-alkyl)amino, di(C₁-C₃-alkyl)amino;
R⁹ is H or C₁-C₃-alkyl which can be unsubstituted or carry one or more substituents from the group consisting of OH, C(O)H, C(O)CH₃, C(O)C₂H₅, halogens, pseudohalogens, NH₂, mono(C₁-C₃-alkyl)amino, di(C₁-C₃-alkyl)amino;
R¹⁰, R¹¹ independently have the same meaning as R³, R⁴;
R¹², R¹³ independently are H or unsubstituted and at least monosubstituted C₁-C₁₂-alkyl which can carry in its chain one or more non-adjacent heteroatoms from the group nitrogen and oxygen, and which, if substituted, carry one or more substituents from the group consisting of: OH, C(O)H, C(O)CH₃, C(O)C₂H₅, halogens, pseudohalogens, NH₂, mono(C₁-C₃-alkyl)amino, di(C₁-C₃-alkyl)amino, and unsubstituted and at least monosubstituted aryl and heteroaryl, which, if substituted, carry at least one substituent from the group consisting of C₁-C₃-alkyl, C₁-C₃-alkoxy, halogens, pseudohalogens, and CF₃;
R¹⁴ has the same meaning as R¹²
R¹⁵, R¹⁶ independently have the same meaning as R¹², R¹³;
R¹⁷ has the same meaning as R¹²;
R¹⁸ has the same meaning as R¹²
R¹⁹, R²⁰ independently have the same meaning as R¹², R¹³;
R²¹ has the same meaning as R¹²;
R²² has the same meaning as R¹²;
aryl is 5 to 10-membered, mono- or bicyclic aromatic cycle;
heteroaryl is a 5 to 10-membered, mono- or bicyclic aromatic heterocycle containing one or more heteroatoms from the group consisting of N, O and S;
mis 1, 2 or 3.

Alkyl residues can be linear or branched, acyclic or cyclic. This also applies when they are part of other groups, for example in alkoxy groups, alkoxycarbonyl groups or amino groups, or when they are substituted.

Examples for alkyl groups are methyl, ethyl, propyl, butyl, pentyl, hexyl, the n-isomers of these residues, isopropyl, isobutyl, isopentyl, sec-butyl, tert-butyl, neopentyl, 3,3-dimethylbutyl. The term alkyl here also expressly includes cycloalkyl residues and cycloalkyl-alkyl-residues (alkyl substituted by cycloalkyl) containing at least three carbon atoms. Examples for such cycloalkyl residues are cyclopropyl, cyclobutyl, cyclopentyl, cyclohexyl. All cycloalkyl groups can be substituted by one or more identical or different (C₁-C₄)-alkyl residues, in particular by methyl. Furthermore, unless stated otherwise, the term alkyl here also includes unsubstituted alkyl residues as well as alkyl residues which are substituted by one or more, for example one, two, three or four, identical or different residues, for example aryl groups. In substituted alkyl residues, for example arylalkyl, hydroxyalkyl such as -(C₁-C₃)-alkyl-OH or alkoxyalkyl such as -(C₁-C₃)-alkyl-O-(C₁-C₄)-alkyl, the substituents can be present in any desired position.

The term methylene group as used in the context of the present invention denotes a -CH₂- unit or a unit derived therefrom by replacing one or both H atom for a substituent.

The term aryl as used in the context of the present invention comprises mononuclear, binuclear or trinuclear aryl groups having no heteroatoms in its cycle. If not stated otherwise, the aryl groups can carry one or more substituents for example alkyl groups, alkoxy groups, halogens or OH groups. Examples for aryl groups include phenyl, 1-naphthyl, 2-naphthyl, indanyl, toluyl.

Unless stated otherwise, heteroaryl residues which are formed by two groups bonded to a nitrogen are preferably derived from heterocycles which contain one, two, three or four heteroatoms which can be identical or different; more preferably they are derived from heterocycles which contain one, two, or three, in particular one or two, heteroatoms which can be identical or different. Unless stated otherwise, the heterocycles can be mononuclear or polynuclear, for example mononuclear, binuclear or trinuclear. Preferably they are mononuclear or binuclear. The rings preferably are 5-membered rings, 6-membered rings or 7-membered rings. Examples of mononuclear and binuclear heterocyclic systems from which residues occuring in the compounds of the formula (I) can be derived, are pyrrole, furan, thiophene, imidazole, pyrazole, 1,2,3-triazole, 1,2,4-triazole, 1,3-dioxole, 1,3-oxazole (= oxazole), 1,2-oxazole (= isoxazole), 1,3-thiazole (= thiazole), 1,2-thiazole (= isothiazole), tetrazole, pyridine, pyridazine, pyrimidine, pyrazine, pyran, thiopyran, 1,4-dioxine, 1,2-oxazine, 1,3-oxazine, 1,4-oxazine, 1,2-thiazine, 1,3-thiazine, 1,4-thiazine, 1,2,3-triazine, 1,2,4-triazine, 1,3,5-triazine, 1,2,4,5-tetrazine, azepine, 1,2-diazepine, 1,3-diazepine, 1,4-diazepine, 1,3-oxazepine, 1,3-thiazepine, indole, benzothiophene, benzofuran, benzothiazole, benzimidazole, quinoline, isoquinoline, cinnoline, quinazoline, quinoxaline, phthalazine, thienothiophenes, 1,8-naphthyridine and other naphthyridines, pteridin, or phenothiazine, each of them in saturated form (perhydro form) or in partially unsaturated form (for example in the dihydro form or the tetrahydro form) or in maximally unsaturated form, in case the respective forms are known and stable. Thus, suitable heterocycles also include, for example, the saturated heterocycles pyrrolidine, piperidine, piperazine, morpholine and thiomorpholine. The degree of saturation of heterocyclic groups is indicated in their individual definitions. Unsaturated heterocycles can contain, for example, one, two or three double bonds within the ring system. 5-membered rings and 6-membered rings can in particular also be aromatic.

Halogen is fluorine, chlorine, bromine or iodine, preferably fluorine or chlorine.

Examples for pseudohalogens are CN and N₃, a preferred pseudohalogen is CN.

Preferred compounds of the formula (I) are those compounds in which one or more of the residues contained therein have the meanings given in the following, with all combinations of preferred substituent definitions being a subject of the present invention. With respect to all preferred compounds of the formula (I) the present invention also includes all stereoisomeric forms and mixtures thereof in all ratios, and their pharmaceutically acceptable salts.

Compounds of the formula (I) in which all of the above-mentioned groups have the preferred meanings or the particularly preferred meanings are more preferred with respect to the compounds of formula (I) wherein only one or several of the substituents have the preferred or more preferred meanings.

R¹ and R² are independently from each other selected from the group consisting of:
H; OH; (=O); halogens; pseudohalogens; NH₂; S(O)ₘR⁵; SO₂NH₂; C(O)R⁸; C(O)OR⁹; CONH₂; C₁-C₂-alkyl substituted by NN₂, OH, S(O)ₘR⁵, SO₂NH₂, C(O)R⁸, C(O)OR⁹, CONH₂; C₁-C₂-alkoxy substituted by NR₂, OH, S(O)ₘR⁵, SO₂NH₂, C(O)R⁸, C(O)OR⁹, CONH₂;
Ar denotes an unsubstituted mononuclear aryl group having 6 or 7 members, which aryl group is annulated to the neighbouring 5-membered cycle, and which may carry 1, 2 or 3 heteroatoms from the group N, O and S in its cycle;
Y, Z denote independently from each other a nitrogen atom or a methylene group;
X is a nitrogen atom or a methylene group;
A is selected from the group consisting of: H; halogens and pseudohalogens; OH; =N(OH); NR¹²R¹³ ; OSO₃⁻; S(O)ₘR¹⁴; SO₂NR¹⁵R¹⁶; C(O)R¹⁷; C(O)OR¹⁸; CONR¹⁹R²⁰; C(S)R²¹, C(S)OR²²; unsubstituted and at least monosubstituted C₁-C₆-alkyl which can carry in its chain one or more non-adjacent heteroatoms from the group nitrogen and oxygen, and which, if substituted, carry at least one substituent which is preferably selected from the group consisting of: halogens, pseudohalogens, OH, NR¹²R¹³, OSO₃-, S(O)ₘR¹⁴, SO₂NR¹⁵R¹⁶, C(O)R¹⁷, C(O)OR¹⁸, CONR¹⁹R²⁰, C(S)R²¹, C(S)OR²², and substituted and non-substituted aryl and substituted and non-substituted heteroaryl which, if substituted, carry at least one substituent from the group consisting of C₁-C₃-alkyl, C₁-C₃-alkoxy, halogens, pseudohalogens, and CF₃;
R⁵ is selected from H; substituted and unsubstituted methyl and ethyl which, if substituted, can carry one or more substituents from the group OH, halogens, pseudohalogens,;
R⁸ is H or C₁-C₃-alkyl which can be unsubstituted or carry one or more substituents from the group consisting of OH, C(O)H, C(O)CH₃, C(O)C₂H₅, halogens, pseudohalogens; NH₂, mono(C₁-C₃-alkyl)amino, di(C₁-C₃-alkyl)amino;
R⁹ is H or C₁-C₃-alkyl which can be unsubstituted or carry one or more substituents from the group consisting of OH, C(O)H, C(O)CH₃, C(O)C₂H₅, halogens, pseudohalogens; NH₂, mono(C₁-C₃-alkyl)amino, di(C₁-C₃-alkyl)amino;
R¹⁰, R¹¹ independently have the same meaning as R³, R⁴;
R¹², R¹³ independently are H or unsubstituted and at least monosubstituted C₁-C₁₂-alkyl which can carry in its chain one or more non-adjacent heteroatoms from the group nitrogen and oxygen, and which, if substituted, carry one or more substituents from the group consisting of: OH, C(O)H, C(O)CH₃, C(O)C₂H₅, halogens, pseudohalogens, NH₂, mono(C₁-C₃-alkyl)amino, di(C₁-C₃-alkyl)amino, and unsubstituted and at least monosubstituted aryl and heteroaryl, which, if substituted, carry at least one substituent from the group consisting of C₁-C₃-alkyl, C₁-C₃-alkoxy, halogens, pseudohalogens, and CF₃;
R¹⁴ has the same meaning as R¹²
R¹⁵, R¹⁶ independently have the same meaning as R¹², R¹³;
R¹⁷ has the same meaning as R¹²;
R¹⁸ has the same meaning as R¹²
R¹⁹, R²⁰ independently have the same meaning as R¹², R¹³;
R²¹ has the same meaning as R¹²;
R²² has the same meaning as R¹²;
aryl is phenyl, naphth-1-yl or naphth-2-yl.
heteroaryl is preferably selected from the group consisting of 5- and 6- membered monocyclic and 9- or 10-membered bicyclic heterocycles containing one or more heteroatoms from the group consisting of N, O, and S; heteroaryl is in particular selected from the group consisting of indolyl, furyl, pyrrolyl, thienyl, thiazolyl, oxazolyl, pyrazolyl, imidazolyl, pyrazinyl, pyridyl and pyrimidinyl.;
m is 1, 2 or 3.

In a further preferred embodiment of the present invention, at least one substituent R¹ or R² in formula (I) is (=O).

In a further preferred embodiment of the present invention, in case A is a substituted alkyl group, the carbon atom in α-position to X carries at least 1 H-atom.

The object of the present invention is also attained by compounds according to the general formula (I) 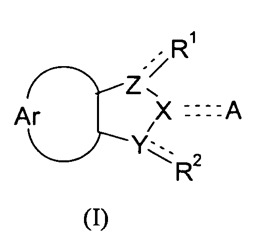 wherein the symbols Ar, A, X, Y, and Z and the substituents R¹ and R² have the meaning defined above, for use as a pharmaceutical.

Preferred compounds of the formula (I) for use as a pharmaceutical are those compounds in which one or more of the residues contained therein have the meanings given above as being preferred, with all combinations of preferred substituent definitions being a subject of the present invention. With respect to all preferred compounds of the formula (I) for use as a pharmaceutical, the present invention also includes all stereoisomeric forms and mixtures thereof in all ratios, and their pharmaceutically acceptable salts.

Compounds of the formula (I) in which all of the above-mentioned groups have the preferred meanings or the particularly preferred meanings are more preferred for use as a pharmaceutical with respect to the compounds of formula (I) wherein only one or several of the substituents have the preferred or more preferred meanings.

The object of the present invention is furthermore attained by the use of a compound of formula (I) 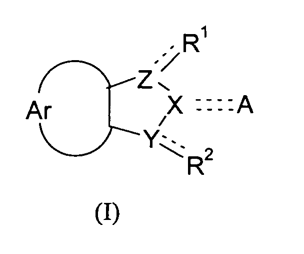 wherein the symbols Ar, A, X, Y, and Z and the substituents R¹ and R² have the meanings defined above, for the manufacture of a pharmaceutical for the inhibition of DNMTs, more particularly DNMT1, and/or the inhibition of DNA methylation, and the pharmaceuticals obtained accordingly.

Preferred compounds of the formula (I) which can be used for the manufacture of the said pharmaceuticals are those compounds in which one or more of the residues contained therein have the meanings given above as being preferred, with all combinations of preferred substituent definitions being a subject of the present invention. With respect to all preferred compounds of the formula (I) which can be used for the manufacture of the said pharmaceuticals are, the present invention also includes all stereoisomeric forms and mixtures thereof in all ratios, and their pharmaceutically acceptable salts.

Compounds of the formula (I) in which all of the above-mentioned groups have the preferred meanings or the particularly preferred meanings are more preferred for the manufacture of the said pharmaceuticals with respect to the compounds of formula (I) wherein only one or several of the substituents have the preferred or more preferred meanings.

The present invention also relates to the use compounds according to formula (I) 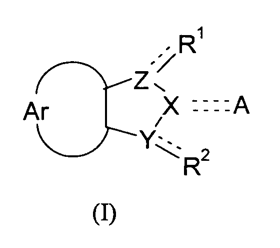 wherein the symbols Ar, A, X, Y, and Z and the substituents R¹ and R² have the meanings defined above, in methods of treatment.

The present invention includes all stereoisomeric forms of the compounds of the formula (I). Centers of asymmetry that are present in the compounds of formula (I) all independently of one another have S configuration or R configuration. The invention includes all possible enantiomers and diastereomers and mixtures of two or more stereoisomers, for example mixtures of enantiomers and/or diastereomers, in all ratios. Thus, compounds according to the present invention which can exist as enantiomers can be present in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. All these forms are an object of the present invention. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at the stage of the compounds of the formula (I) or at the stage of an intermediate during the synthesis. The present invention also includes all tautomeric forms of the compounds of formula (I).

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts.

The present invention furthermore includes all solvates of compounds of the formula (I), for example hydrates or adducts with alcohols, active metabolites of the compounds of the formula (II), and also derivatives and prodrugs of the compounds of the formula (I) which contain physiologically tolerable and cleavable groups, for example esters, amides and compounds in which the N-H group depicted in formula (I) is replaced with an N-alkyl group, such as N-methyl, or with an N-acyl group, such as N-acetyl or N-argininyl, including pharmaceutically acceptable salts formed on functional groups present in the N-acyl group.

The compounds according to general formula (I) and their precursors can be prepared according to methods published in the literature or, respectively, analogous methods. Appropriate methods have been published in, for example, Houben-Weyl, Methoden der Organischen Chemie (Methods of Organic Chemistry), Thieme-Verlag, Stuttgart, or Organic Reactions, John Wiley & Sons, New York.

All reactions for the synthesis of the compounds of the formula (I) are per se well-known to the skilled person and can be carried out under standard conditions according to or analogously to procedures described in the literature, for example in Houben-Weyl, Methoden der Organischen Chemie (Methods of Organic Chemistry), Thieme-Verlag, Stuttgart, or Organic Reactions, John Wiley & Sons, New York. Depending on the circumstances of the individual case, in order to avoid side reactions during the synthesis of a compound of the formula (I), it can be necessary or advantageous to temporarily block functional groups by introducing protective groups and to deprotect them in a later stage of the synthesis, or introduce functional groups in the form of precursor groups which in a later reaction step are converted into the desired functional groups. Such synthesis strategies and protective groups and precursor groups which are suitable in an individual case are known to the skilled person. If desired, the compounds of the formula (I) can be purified by customary purification procedures, for example by recrystallization or chromatography. The starting compounds for the preparation of the compounds of the formula (I) are commercially available or can be prepared according to or analogously to literature procedures. The compounds obtained with the above-mentioned synthesis methods are a further object of the present invention.

A part of the compounds falling under formula (I) are disclosed in the literature. However, their use in inhibition of DNA methylation or the treatment of developmental or proliferative disorders is not disclosed in any of these references.

The compounds according to the general formula (I) can be used to inhibit DNA methylation in cells. Particularly, said compounds inhibit DNMTs, more particularly DNMT1, even more particularly human DNMT1.

As the compounds according to the general formula (I) are inhibitors of DNA methylation, they are helpful pharmaceutical compounds for the treatment of diseases associated with aberrant DNA methylation or treatable by inhibition of DNA methylation. In the context of the present invention, treatment includes the therapy as well as the prophylaxis of the respective diseases.

Aberrant DNA methylation preferably relates to any kind of hypermethylation, be it genome-wide or limited to distinct genomic or chromosomal regions or genes. DNA methylation can be measured by any of the methods known in the art (e.g. Okamoto, A., et al. (2002). Site-specific discrimination of cytosine and 5-methylcytosine in duplex DNA by peptide nucleic acids. J Am. Chem. Soc. 124, 10262-10263), methylation sensitive arbitrarily primed PCR (Gonzalgo, M.L., Liang, G., et al. (1997). Identification and characterization of differentially methylated regions of genomic DNA by methylation-sensitive arbitrarily primed PCR. Cancer Res. 57:594-599), methylated CpG island amplification (Toyota, M., Ho, C., et al. (1999). Identifation of differentially methylated sequences in colorectal cancer by methylated CpG island amplification. Cancer Res. 59:2307-2312), restriction landmark genomic scanning (RLGS) (Hayashizaki, Y., Hirotsune, S., et al., (1993). Restriction landmark genomic scanning method and its various applications. Electrophoresis 14:251-258), differential methylation hybridization (Huang, T. H., Perry, M. R., et al. (1999). Methylation profiling of CpG islands in human breast cancer cells. Hum. Mol. Genet. 8: 459-470), capillary electrophoresis (Stach, D., Schmitz, O.J., Stilgenbauer, S., et al. 2003. Nucleic Acids Res. 31, e2), and microarray-based techniques (Adorjan, P., Distler, J., et al. (2002). Tumour class prediction and discovery by microarray-based DNA methylation analysis. Nucleic Acids Research 30(5): e21). Thus, it is also possible to identify diseases associated with aberrant DNA methylation.

Examples of diseases which can be treated with the compounds according to the present invention include developmental disorders and proliferative diseases.

Examples for developmental disorders which can be treated with the compounds according to the present invention include Prader-Willi-Syndrome, Angelman-Syndrome (Happy Puppet Syndrome), and Beckwith-Wiedemann-Syndrome.

Examples for proliferative diseases which can be treated with the compounds according to the present invention include coronary restenosis and neoplastic diseases.

Said neoplastic diseases include neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, prostate carcinoma, and plasmocytoma.

Preferred indications are colon carcinoma, familiary adenomatous polyposis carcinoma and hereditary non-polyposis colorectal cancer, prostate carcinoma, melanoma, non-Hodgkin lymphoma, acute lymphatic leukemia (ALL), chronic lymphatic leukemia (CLL), acute myeolid leukemia (AML), chronic myeloid leukemia (CML), or hepatocellular carcinoma.

The compounds according to the formula (I) can also be used in combination with other pharmaceutically active compounds, preferably compounds which are able to enhance the effect of the compounds according to the general formula (I). Examples of such compounds include: (i) antimetabolites, cytarabine, fludarabine, 5-fluoro-2'-deoxyuridine, gemcitabine, hydroxyurea or methotrexate; (ii) DNA-fragmenting agents, bleomycin, (iii) DNA-crosslinking agents, chlorambucil, cisplatin, fotemustine, cyclophosphamide or nitrogen mustard; (iv) intercalating agents, adriamycin (doxorubicin) or mitoxantrone; (v) protein synthesis inhibitors, L-asparaginase, cycloheximide, puromycin or diphteria toxin; (vi) topoisomerase I poisons, camptothecin or topotecan; (vii) topoisomerase II poisons, etoposide (VP-16) or teniposide; (viii) microtubule-directed agents, colcemid, colchicine, paclitaxel (taxol), docetaxel (taxotere), vinblastine or vincristine; (ix) kinase inhibitors, flavopiridol, staurosporin, STI571 (CPG 57148B) or UCN-01 (7-hydroxystaurosporine); (x) miscellaneous investigational agents, trichostatin A, thioplatin, PS-341, phenylbutyrate, ET-18-OCH₃, or farnesyl transferase inhibitors (L-739749, L-744832); polyphenols, quercetin, resveratrol, piceatannol, epigallocatechine gallate, theaflavins, flavanols, procyanidins, betulinic acid and derivatives thereof; (xi) hormones, glucocorticoids or fenretinide; (xii) hormone antagonists, tamoxifen, finasteride or LHRH antagonists, (xiii) demethylating agents, 5-azacytidine, 5-aza-2'deoxycytidine, 5,6-dihydro-5-azacytidine, or (xiv) a combination of any of the pharmaceuticals given above.

Preferred compounds are the compounds according to the formulae below.

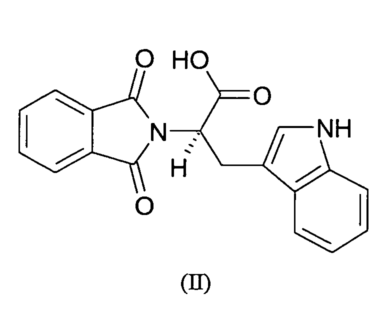

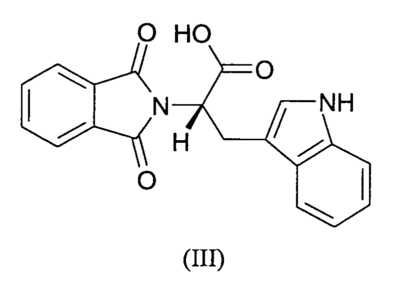

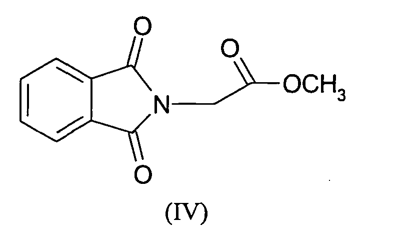

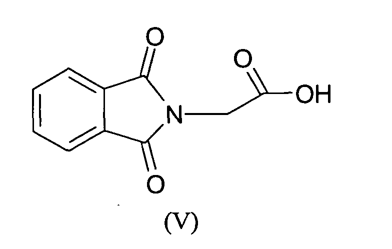

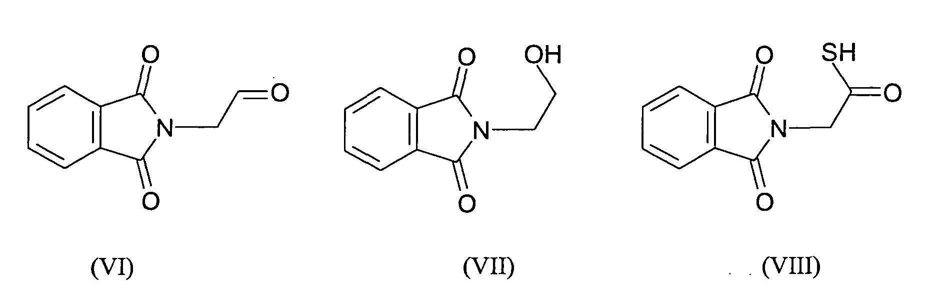

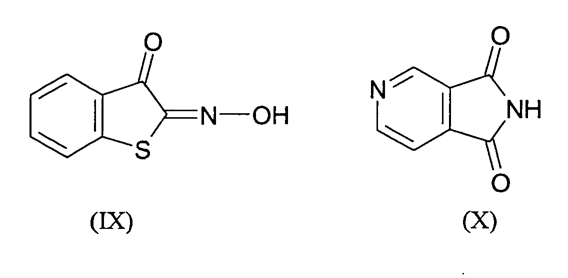

The compounds of the formula (I) and their pharmaceutically acceptable salts, optionally in combination with other pharmaceutically active compounds, can be administered to animals, preferably to mammals, and in particular to humans, as pharmaceuticals by themselves, in mixtures with one another or in the form of pharmaceutical preparations. Further subjects of the present invention therefore also are the compounds of the formula (I) and their pharmaceutically acceptable salts for use as pharmaceuticals, their use as inhibitors of DNMTs and/or DNA methylation, and in particular their use in the therapy and prophylaxis of the above-mentioned syndromes as well as their use for preparing pharmaceuticals for these purposes. Furthermore, subjects of the present invention are pharmaceutical preparations (or pharmaceutical compositions) which comprise an effective dose of at least one compound of the formula (I) and/or a pharmaceutically acceptable salt thereof and a pharmaceutically acceptable carrier, i.e. one or more pharmaceutically acceptable carrier substances and/or additives.

The pharmaceuticals according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The amount of compounds of the formula (I) and/or its pharmaceutically acceptable salts in the pharmaceutical preparations normally ranges from 0.2 to 800 mg, preferably from 0.5 to 500 mg, in particular from 1 to 200 mg, per dose, but depending on the type of the pharmaceutical preparation it may also be higher. The pharmaceutical preparations usually comprise 0.5 to 90 percent by weight of the compounds of the formula (I) and/or their pharmaceutically acceptable salts. The preparation of the pharmaceutical preparations can be carried out in a manner known per se. To this end, one or more compounds of the formula (I) and/or their pharmaceutically acceptable salts, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiologically sodium chloride solution, alcohols such as ethanol, glycerol, polyols, sucrose, invert sugar, glucose, mannitol, vegetable oils, etc. It is also possible to lyophilize the compounds of the formula (I) and their pharmaceutically acceptable salts and to use the resulting lyophilisates, for example, for preparing preparations for injection or infusion. Suitable carriers for microcapsules, implants or rods are, for example, copolymers of glycolic acid and lactic acid.

Besides the compound or compounds according to the invention and carriers, the pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the compound of the formula (I) to be administered and/or of a pharmaceutically acceptable salt thereof depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to compounds of the formula (I). In general, a daily dose of approximately 0.01 to 100 mg/kg, preferably 0.1 to 10 mg/kg, in particular 0.3 to 5 mg/kg (in each case mg per kg of bodyweight) is appropriate for administration to an adult weighing approximately 75 kg in order to obtain the desired results. The daily dose can be administered in a single dose or, in particular when larger amounts are administered, be divided into several, for example two, three or four individual doses. In some cases, depending on the individual response, it may be necessary to deviate upwards or downwards from the given daily dose.

The compounds of the formula (I) may also be used to induce cellular differentiation in vivo and in vitro. Cellular differentiation relates to any differentiation of a cell from a less differentiated (specialized) state to a more differentiated (specialized) state. Cell types which can be treated include, but are not limited to, embryonic and adult stem cells, totipotent, omnipotent, pluripotent, multipotent, oligopotent, or monopotent stem cells, progenitor cells, committed progenitor cells, as well as stem cells derived from bone marrow, peripheral blood, umbilical cord blood, adipose tissue, heart muscle, intestine, small intestine, or brain. Particular examples include multipotent adult progenitor cells (MAPCs), mesenchymal stem cells, hematopoetic stem cells, intestinal stem cells, hepatic stem cells (oval cells), neuronal stem cells, epidermal stem cells, myoblasts, cardiomyoblasts, osteoblasts, chondroblasts, and basal cells of epithelia, e.g. the respiratory epithelium.

The compounds according to formula (I) are capable of binding to DNMTs, particularly human DNMT1, and inhibiting their catalytic activity.

The GenBank accession numbers of selected DNMTs of human, mouse, Drosphila melanogaster, Haemophilus haemolyticus and Haemophilus aegyptius are given below:
human DNMT1 protein (GenBank Acc. No. NP_001370)
human DNMT2 protein (GenBank Acc. No. AAC39764)
human DNMT3A protein (GenBank Acc. No. AAD33084)
human DNMT3B protein (GenBank Acc. No. AAD53063)
mouse DNMT1 protein (GenBank Acc. No. NP_034196)
D. melanogaster dDNMT2 protein (GenBank Acc. No. AAF03835)
H. haemolyticus M.HhaI methyltransferase protein (GenBank Acc. No. XYHIH1)
H. aegyptius M.HhaI methyltransferase protein (GenBank Acc. No. AAA24970)

The present invention explicitly includes the use of the compounds according to formula (I) for inhibition of DNA methylation or inhibition of DNMTs in cells of prokaryotes, eukaryotes, invertebrates, vertebrates, particularly mammals, more particularly rodents and primates, even more particularly humans.

Different DNMTs in different species are highly conserved, i.e. structurally similar, particularly with respect to their C-terminal catalytic domains (see SEQ ID No. 1-8). Therefore, the compounds according to formula (I) are capable of binding and inhibiting different DNMTs in different species.

### Figure legends

### Fig. 1

Calculated binding energies of NSC401077 and various control molecules docked into the DNMT1 active site (a). The calculated binding energy of NSC401077 exceeds that of the natural substrate, cytidine. (b) Structure of RG108. The compound was synthesized as an L-enantiomer, while NSC401077 represents a racemic mixture of both enantiomers.

### Fig. 2

Direct inhibition of DNA methyltransferase activity by RG108. (a) In vitro methylation assay. Equal amounts of purified SssI methylase were incubated with a DNA fragment derived from the human p16/CDKN2A promoter and 200 µM RG108. This resulted in a detectable decrease in DNA methyltransferase activity, as visualized by the appearance of small *Bst*UI restriction fragments. No inhibitory effect was observed with equal concentrations of 5-azacytidine. (b) Increasing amounts of RG108 progressively inhibit methyltransferase activity. RG108 was used at 0, 10, 200, and 500 µM concentrations, SssI methylase was present at 5 µM concentration. (c) Trapping assay. HCT116 cells were incubated with equal concentrations of RG108, 5-azacytidine, or no inhibitor. Protein extracts were then probed for the presence of DNMT1, DNMT3B and Actin by Western blotting. This revealed the covalent trapping of DNA methyltransferase proteins by 5-azacytidine. This effect was not observed with RG108.

### Fig. 3

The effect of RG108 on human cell lines. (a) Growth of HCT116 (left panel) and NALM-6 cells (right panel) in medium supplemented with 10 µM RG 108 (filled circles) or 10 µM 5-azacytidine (grey diamonds). Controls are shown as open squares. (b) Viability of HCT116 and NALM-6 cells under the same conditions as described in (a). (c) Genomic cytosine methylation levels of HCT116 (left panel) and NALM-6 cells (right panel)) in medium supplemented with 10 µM RG 108 (black bars) or 10 µM 5-azacytidine (grey bars). Controls are shown as white bars. DNA methylation levels were determined after 5 and 15 days, as indicated. DNA from NALM-6 cells incubated with 5-azacytidine for 15 days could not be analyzed due to degradation. All results were obtained from multiple experiments. Standard deviations for panels (a) and (b) were negligible.

### Fig. 4

The carboxyl-group of RG108 is important for its interaction with DNA methyltransferases. (a) Structure of ΔC-RG108, a control compound that lacks the carboxyl-group of RG-108. (b) Calculated binding energies of ΔC-RG108 (grey bar) docked into the DNMT1 active site are compared to cytidine (white bar) and RG108 (black bar). (c) Genomic cytosine methylation levels of HCT116 cells incubated with ΔC-RG108 (black bar) are compared to methylation levels of corresponding cells incubated with no inhibitor (white bar) or RG108 (grey bar).

### Fig. 5

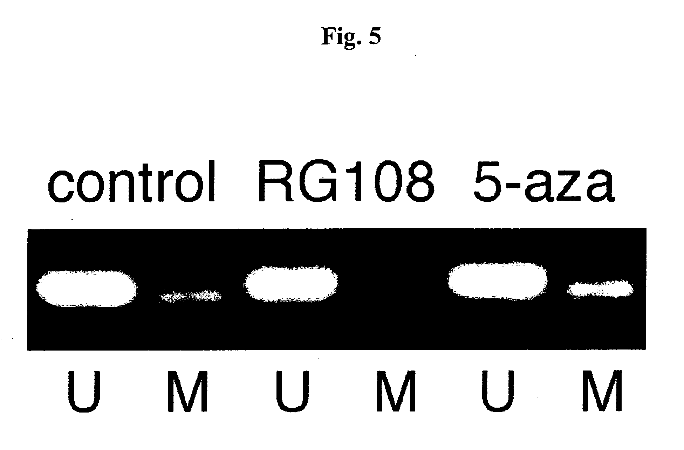

RG108 causes complete demethylation of the human hMLH1 gene in cells treated with 10 micromolar RG108, while no demethylation was observed in control experiments without inhibitor or with 10 micromolar 5-azacytidine (Sigma), respectively (Fig. 5). M indicates amplification products from methylated templates, U indicates amplification from unmethylated control templates.

The present invention will now be illustrated in the following examples:

### EXAMPLES

### Example 1

Chemical synthesis. RG108 was synthesized in two steps with an overall yield of 90%. The intermediate product, a phthalic acid derivative with a protected and an activated ester group, methyl 2-((succinimidooxy)carbonyl)benzoate (MSB) was obtained according to Casimir, J.R., Guichard, G. & Briand, J.P. Methyl 2-((succinimidooxy)carbonyl)benzoate (MSB): a new, efficient reagent for N-phthaloylation of amino acid and peptide derivatives. J. Org. Chem. 67, 3764-3768 (2002) from 2-(methoxycarbonyl)benzoic acid and N-hydroxysuccinimide in 90% yield after crystallisation and drying. The structure was confirmed by mass spectrometry (ESI) and ¹H- and ¹³C-NMR analysis. RG108 was obtained by the reaction of MSB with L-tryptophan under basic conditions (Na₂CO₃) in water/acetonitrile, acidification with 2N HCl, extraction in ethyl acetate and evaporation of the solvent with an excellent yield of 100%. The pure yellow powder was analysed by mass spectrometry (ESI) and 1H- and ¹³C-NMR to confirm the structure of RG108. For the synthesis of ΔC-RG108, tryptamine (dissolved in acetonitrile) was used instead of L-tryptophan.

High performance liquid chromatography analysis indicated that RG108 had a half-life time of 10 days in neutral aqueous solutions at 37 °C.

### Example 2

Analysis of genomic DNA methylation. DNA was isolated from cells using the DNeasy kit from Qiagen. Cytosine methylation levels were determined by capillary electrophoresis, as described previously (Stach, D., Schmitz, O.J., Stilgenbauer, S., et al. 2003. Nucleic Acids Res. 31, e2).

### Example 3

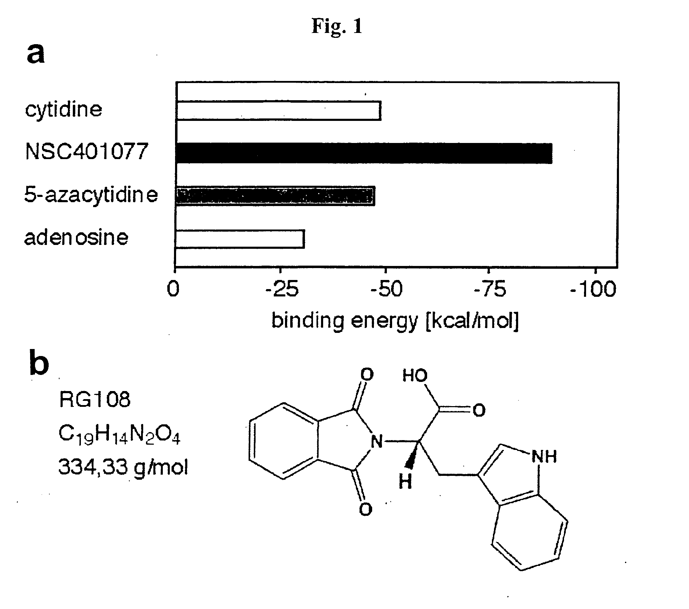

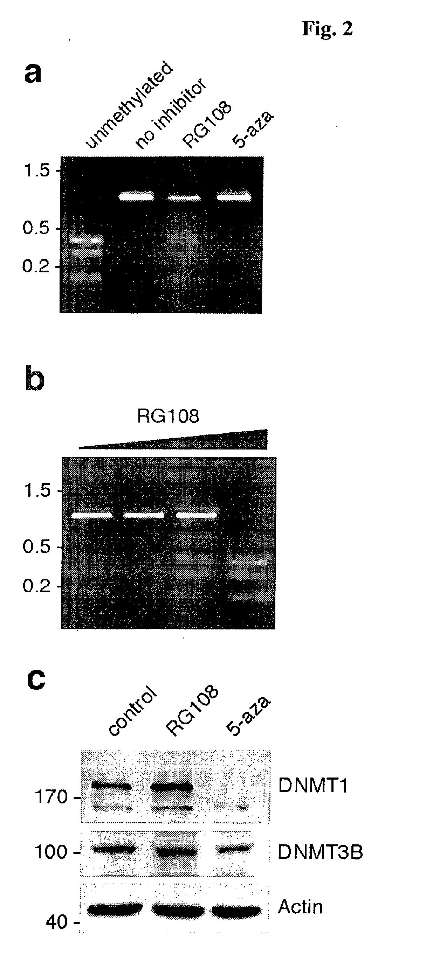

The effect of RG108 was analyzed in an in vitro DNA methylation assay. For this assay, the purified recombinant CpG methylase M.SssI was used. This enzyme is distinguished by a robust activity and also shows significant structural similarities with the DNMT1 catalytic domain. A 798 bp PCR fragment from the promoter region of the human p16/CDKN2A gene was used as a substrate and DNA methylation was visualized by digestion with the methylation-sensitive restriction enzyme *Bst*UI. Thus, inhibition of DNA methyltransferases can be detected by the appearance of unprotected, smaller restriction fragments (Fig. 2a). A direct comparison of RG108 and 5-azacytidine in this assay revealed a readily detectable inhibitory effect for RG108, but not for 5-azacytidine (Fig. 2a). This result is consistent with the assumption that RG108 is able to inhibit the free DNA methyltransferase, while 5-azacytidine needs to be incorporated into DNA. Additional experiments showed that increasing concentrations of RG108 resulted in increasing amounts of unprotected *Bst*UI restriction fragments (Fig. 2b) and thus confirmed a significant, concentration-dependent inhibition by RG108. Complete inhibition was observed when the molar ratio of RG108 to SssI methylase was roughly 100:1 (Fig. 2b).

### Example 4

Inhibitors like 5-azacytidine and zebularine, have been shown to covalently trap DNA methyltransferases, which can can be visualized by concomitant depletion of the enzymes from cell extracts (Liu, K., Wang, Y.F., Cantemir, C. & Muller, M.T. (2003). Endogenous assays of DNA methyltransferases: Evidence for differential activities of DNMT1, DNMT2, and DNMT3 in mammalian cells In vivo. *Mol. Cell. Biol.* **23**, 2709-2719 and Cheng, J.C. et al. (2004). Continuous zebularine treatment effectively sustains demethylation in human bladder cancer cells. *Mol Cell Biol* **24**, 1270-1278.

To characterize the inhibitory mechanism of RG108 human HCT116 cells were incubated with RG108 or an equal concentration of 5-azacytidine and protein extracts were analyzed for the presence of the major DNA methyltransferases, DNMT1 and DNMT3B, and actin (as a control). This demonstrated that DNA methyltransferase levels were not affected by RG108 (Fig. 2c), which suggested that RG108 does not result in covalent trapping of DNA methyltransferases. In contrast, 5-azacytidine resulted in a strong depletion of DNMT1 and a weaker reduction in DNMT3B levels (Fig. 2c). These results are consistent with the direct inhibition observed in vitro and provide a major distinction between RG108 and previously known inhibitors of DNA methyltransferases.

### Example 5

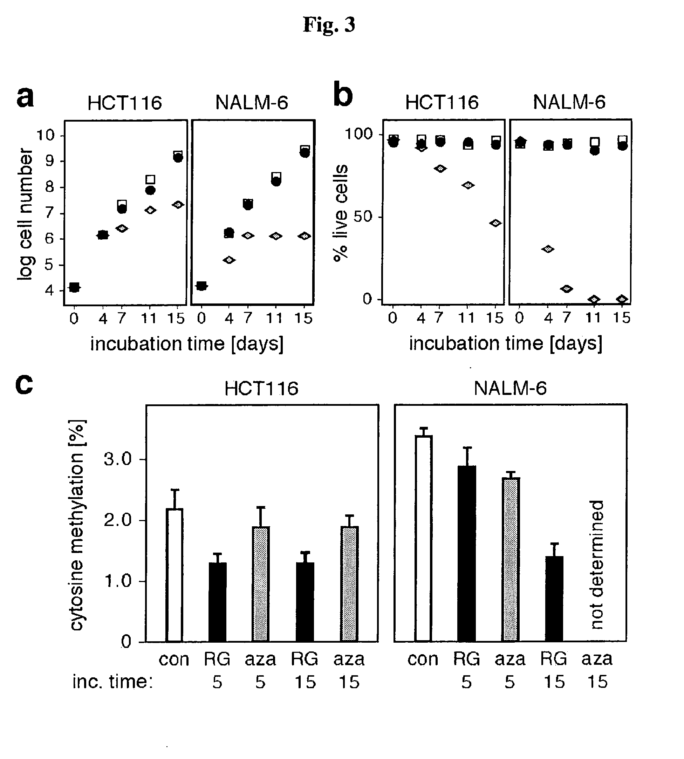

The effect of RG108 on the growth and viability of human cell lines was characterized. HCT116 was chosen, a colon carcinoma line that has been frequently used for DNA methylation analysis (Brattain, M.G., Fine, W.D., Khaled, F.M., Thompson, J. & Brattain, D.E. Heterogeneity of malignant cells from a human colonic carcinoma. *Cancer Res* **41**, 1751-1756 (1981)), and NALM-6, a leukemic B cell precursor line (Hurwitz, R. et al. Characterization of a leukemic cell line of the pre-B phenotype. *Int. J. Cancer* ,**23** 174-180 (1979)). The tissue culture media was supplemented with 10 µM RG108 and the cells were incubated over 15 days. Unsupplemented media and media supplemented with 10 µM 5-azacytidine were used for controls. At this concentration, RG108 had no effect on the growth and viability of either cell line (Fig. 3a, b). In contrast, 5-azacytidine showed an intermediate effect on HCT116 cells and appeared to be highly toxic for NALM-6 cells (Fig. 3a, b). To analyze the effect of RG108 on DNA methylation, genomic DNA was isolated from cells and the cytosine methylation level was determined by capillary electrophoresis. This revealed a significant (30 %) demethylation of genomic DNA in HCT116 cells that was similar to the effect seen with equal concentrations of 5-azacytidine (Fig. 3c). Significant demethylation of genomic DNA was also observed in NALM-6 cells, where incubation with RG108 reduced DNA methylation by 15 % (after 5 days) and 60 % (after 15 days), respectively (Fig. 3c). Because of the high toxicity of 5-azacytidine in NALM-6 cells, the corresponding DNA methylation level (5 days, Fig. 3c) needs to be interpreted with caution. After 15 days of incubation in 5-azacytidine containing medium, the DNA from NALM-6 cells appeared to be degraded and was not used for further analysis. In summary, the results suggest that RG108 effectively inhibits DNA methylation in human cell lines. At the same time, incubation with RG108 did not seem to affect the growth and viability of these cells under the conditions used in our experiments.

### Example 6

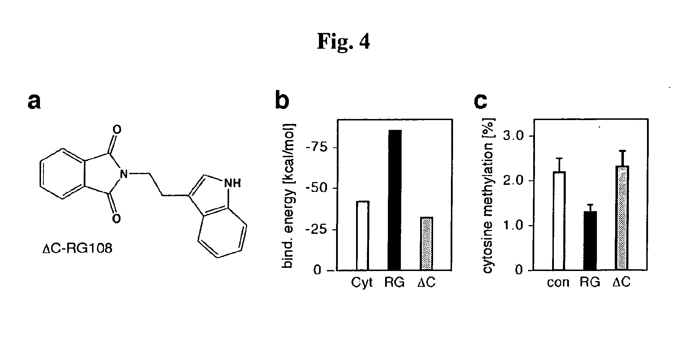

Also derivatives of RG108 with a potentially reduced inhibitory activity were investigated. ΔC-RG108 was synthesized, which is a derivative that lacks the central carboxyl-group (Fig. 4a). Docking of this compound into the DNMT1 active site revealed a strongly reduced binding energy (Fig. 4b). When tested in the in vitro assay, ΔC-RG108 failed to inhibit DNA methylation. Similarly, ΔC-RG108 failed to demethylate genomic DNA of HCT116 and NALM-6 cells (Fig. 4c). The results confirm an important role for the carboxyl-group of RG108 in the interaction with the active site of the enzyme and suggest a considerable specificity in the interaction between RG108 and the DNA methyltransferase active site.

### Example 7

The substrate DNA for the in vitro methylation assay was generated by PCR amplification of a 798 bp fragment from the promoter region of the huma p16/CDKN2 gene. The methylation reaction contained 350-400 ng substrate DNA (13-15 nM DNA, corresponding to 1.6-1.8 µM CpGs) in reaction buffer (50 mM NaCl, 10 mM Tris-HCl, 10 mM MgCl₂, 1mM dithiothreitol, pH 7.9), 80 µM S-adenosylmethionine, and 4 U of M.SssI methylase (0.5 µM, New England Biolabs) in a final volume of 50 µl. Inhibitors were added in concentrations of 10, 100, 200, and 500 µM, respectively. Reactions were performed at 37 °C for 2 hours. After completion, the reaction was inactivated at 65 °C for 15 min and the DNA was purified using the QIAquick PCR Purification Kit (Qiagen). 250 ng of purified DNA was digested for 3 h at 60 °Cwith 30 units of BstUI (New England Biolabs) and analyzed on 2 % agarose gels.

### Example 8

Trapping assay: Frozen cell pellets (10⁶-10⁸ cells) were thawed on ice and resuspended in 1 ml ice-cold 1x PBS. After centrifugation (< 1000g) at 4 °C for 5 minutes, the supernatant was removed and discarded. The pellet was resuspended with 100-200 µl ice-cold lysis buffer (150 mM NaCl, 5 mM EDTA, 50 mM TrisHCl (pH 8.0), 2 mM PMSF, and 1 % Igepal) and incubated on ice for 40 minutes. The lysate was centrifuged at 4 °C for 15 minutes with 14,000 rpm. Supernatants were frozen in liquid nitrogen and stored at -80 °C. Equal amounts of protein were then separated on SDS polyacrylamide gel and analyzed by Western blotting using standard procedures. The primary antibodies used were: anti-DNMT1 (New England Biolabs), 1:2000; anti-DNMT3b (Abgent), 1:250; anti-actin (Abcam), 1:5000. Primary antibodies were visulaized by ECL chemiluminescence (Perkin-Elmer) according to the manufacturer's protocol.

### Example 9

NALM-6 and HCT116 cells were cultured under standard conditions in RPMI 1640 and McCoy's 5a medium, respectively. To analyze the effect of DNA methyltransferase inhibitors, cells were continuously cultivated in media supplemented with 10 µM 5-azacytidine, RG108 or ΔC-RG108, as indicated. Cells were diluted 1:10 in fresh media every 3 or 4 days. For the determination of cellular growth and viability, cells were stained with trypan blue and counted using a standard counting grid.

### Example 10

RG108 causes demethylation in HCT116 cells. We have used methylation-specific PCR (Herman, J.G., Graff, J.R., Myohanen, S., Nelkin, B.D., Baylin, S.B. Methylation-specific PCR: a novel PCR assay for methylation status of CpG islands. Proc. Natl. Acad. Sci. USA. 1996, 93:9821-9826) to determine the effect of RG108 on the methylation of the human hMLH1 gene in HCT116 cells. This revealed a complete demethylation of hMLH1 in cells treated with 10 micromolar RG108, while no demethylation was observed in control experiments without inhibitor or with 10 micromolar 5-azacytidine (Sigma), respectively (Fig. 5).

## Claims

1. A compound according to the general formula 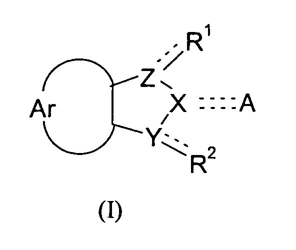 wherein the dotted lines denote a single bond which is optionally present, with 1 dotted line and 1 full line or 2 dotted lines denoting a double bond; and
wherein the symbols have the following meanings:
R¹ and R² are independently from each other selected from the group consisting of:
H; (=O); OH; OSO₃⁻; halogens; pseudohalogens; NR³R⁴; S(O)ₘR⁵; SO₂NR⁶R⁷; C(O)R⁸; C(O)OR⁹; CONR¹⁰R¹¹; substituted and unsubstituted C₁-C₃-alkyl and substituted and unsubstituted C₁-C₃-alkoxy, which alkyl and alkoxy groups, if substituted, carry at least one substituent from the group: OH, OSO₃⁻, halogens, pseudohalogens, NR³R⁴, S(O)ₘR⁵, SO₂NR⁶R⁷, C(O)R⁸, C(O)OR⁹, CONR¹⁰R¹¹, and wherein at least one substituent is different from H;
Ar denotes a substituted or unsubstituted mononuclear aryl group having 6 or 7 members, which aryl group is annulated to the neighbouring 5-membered cycle, and which may carry 1, 2 or 3 heteroatoms from the group N, O and S in its cycle;
Y, Z denote independently from each other a nitrogen atom, an oxygen atom, a sulfur atom or a methylene group;
X is a nitrogen atom, an oxygen atom, a sulfur atom, or a methylene group;
A is selected from the group consisting of: H; halogens and pseudohalogens; OH; =N(OH); NR¹²R¹³; OSO₃⁻; S(O)ₘR¹⁴; SO₂N¹⁵R¹⁶; C(O)R¹⁷; C(O)OR¹⁸; CONR¹⁹R²⁰; C(S)R²¹, C(S)OR²²; unsubstituted and at least monosubstituted C₁-C₁₂-alkyl which can carry in its chain one or more non-adjacent heteroatoms from the group nitrogen and oxygen, and which, if substituted, carry at least one substituent which is preferably selected from the group consisting of: halogens, pseudohalogens, OH, NR¹²R¹³ OSO₃-, S(O)ₘR¹⁴, SO₂NR¹⁵R¹⁶, C(O)R¹⁷, C(O)OR¹⁸, CONR¹⁹R²⁰,
C(S)R²¹, C(S)OR²², and substituted and non-substituted aryl and substituted and non-substituted heteroaryl which, if substituted, carry at least one substituent from the group consisting of C₁-C₃-alkyl, C₁-C₃-alkoxy, halogens, pseudohalogens, and CF₃;
R³, R⁴ are independently selected from the group consisting of:
H; substituted and unsubstituted methyl and ethyl which, if substituted, can carry one or more substituents from the group OH, halogens, pseudohalogens,;
R⁵ independently has the same meaning as R³;
R⁶ and R⁷ independently have the same meaning as R³, R⁴;
R⁸ is H or C₁-C₃-alkyl which can be unsubstituted or carry one or more substituents from the group consisting of OH, C(O)H, C(O)CH₃, C(O)C₂H₅, halogens, pseudohalogens, NH₂, mono(C₁-C₃-alkyl)amino, di(C₁-C₃-alkyl)amino;
R⁹ is H or C₁-C₃-alkyl which can be unsubstituted or carry one or more substituents from the group consisting of OH, C(O)H, C(O)CH₃, C(O)C₂H₅, halogens, pseudohalogens, NH₂, mono(C₁-C₃-alkyl)amino, di(C₁-C₃-alkyl)amino;
R¹⁰, R¹¹ independently have the same meaning as R³, R⁴;
R¹² , R¹³ independently are H or unsubstituted and at least monosubstituted C₁-C₁₂-alkyl which can carry in its chain one or more non-adjacent heteroatoms from the group nitrogen and oxygen, and which, if substituted, carry one or more substituents from the group consisting of: OH, C(O)H, C(O)CH₃, C(O)C₂H₅, halogens, pseudohalogens, NH₂, mono(C₁-C₃-alkyl)amino, di(C₁-C₃-alkyl)amino, and unsubstituted and at least monosubstituted aryl and heteroaryl, which, if substituted, carry at least one substituent from the group consisting of C₁-C₃-alkyl, C₁-C₃-alkoxy, halogens, pseudohalogens, and CF₃;
R¹⁴ has the same meaning as R¹²
R¹⁵, R¹⁶ independently have the same meaning as R¹², R¹³;
R¹⁷ has the same meaning as R¹²;
R¹⁸ has the same meaning as R¹²
R¹⁹, R²⁰ independently have the same meaning as R¹², R¹³;
R²¹ has the same meaning as R¹²;
R²² has the same meaning as R¹²;
aryl is 5 to 10-membered, mono- or bicyclic aromatic cycle;
heteroaryl is a 5 to 10-membered, mono- or bicyclic aromatic heterocycle containing one or more heteroatoms from the group consisting of N, O and S;
m is 1, 2 or 3,
or a pharmaceutically acceptable salt thereof.

2. A compound according to claim 1, wherein the symbols have the following meanings:
R¹ and R² are independently from each other selected from the group consisting of:
H; OH; (=O); halogens; pseudohalogens; NN₂; S(O)ₘR⁵; SO₂NH₂; C(O)R⁸; C(O)OR⁹; CONH₂; C₁-C₂-alkyl substituted by NH₂, OH, S(O)ₘR⁵, SO₂NH₂, C(O)R⁸, C(O)OR⁹, CONH₂; C₁-C₂-alkoxy substituted by NH₂, OH, S(O)ₘR⁵, SO₂NH₂, C(O)R⁸, C(O)OR⁹, CONH₂;
Ar denotes an unsubstituted mononuclear aryl group having 6 or 7 members, which aryl group is annulated to the neighbouring 5-membered cycle, and which may carry 1, 2 or 3 heteroatoms from the group N, O and S in its cycle;
Y, Z denote independently from each other a nitrogen atom or a methylene group;
X is a nitrogen atom or a methylene group;
A is selected from the group consisting of: H; halogens and pseudohalogens; OH; =N(OH); NR¹²R¹³; OSO₃⁻; S(O)ₘR¹⁴; SO₂NR¹⁵R¹⁶; C(O)R¹⁷; C(O)OR¹⁸; CONR¹⁹R²⁰; C(S)R²¹, C(S)OR²²; unsubstituted and at least monosubstituted C₁-C₆-alkyl which can carry in its chain one or more non-adjacent heteroatoms from the group nitrogen and oxygen, and which, if substituted, carry at least one substituent which is preferably selected from the group consisting of: halogens, pseudohalogens, OH, NR¹²R¹³, OSO₃⁻, S(O)ₘR¹⁴, SO₂NR¹⁵R¹⁶, C(O)R¹⁷, C(O)OR¹⁸, CONR¹⁹R²⁰, C(S)R²¹, C(S)OR²², and substituted and non-substituted aryl and substituted and non-substituted heteroaryl which, if substituted, carry at least one substituent from the group consisting of C₁-C₃-alkyl, C₁-C₃-alkoxy, halogens, pseudohalogens, and CF₃;
R⁵ is selected from H; substituted and unsubstituted methyl and ethyl which, if substituted, can carry one or more substituents from the group OH, halogens, pseudohalogens,;
R⁸ is H or C₁-C₃-alkyl which can be unsubstituted or carry one or more substituents from the group consisting of OH, C(O)H, C(O)CH₃, C(O)C₂H₅, halogens, pseudohalogens; NH₂, mono(C₁-C₃-alkyl)amino, di(C₁-C₃-alkyl)amino;
R⁹ is H or C₁-C₃-alkyl which can be unsubstituted or carry one or more substituents from the group consisting of OH, C(O)H, C(O)CH₃, C(O)C₂H₅, halogens, pseudohalogens; NH₂, mono(C₁-C₃-alkyl)amino, di(C₁-C₃-alkyl)amino;
R¹⁰, R¹¹ independently have the same meaning as R³, R⁴;
R¹², R¹³ independently are H or unsubstituted and at least monosubstituted C₁-C₁₂-alkyl which can carry in its chain one or more non-adjacent heteroatoms from the group nitrogen and oxygen, and which, if substituted, carry one or more substituents from the group consisting of: OH, C(O)H, C(O)CH₃, C(O)C₂H₅, halogens, pseudohalogens, NH₂, mono(C₁-C₃-alkyl)amino, di(C₁-C₃-alkyl)amino, and unsubstituted and at least monosubstituted aryl and heteroaryl, which, if substituted, carry at least one substituent from the group consisting of C₁-C₃-alkyl, C₁-C₃-alkoxy, halogens, pseudohalogens, and CF₃;
R¹⁴ has the same meaning as R¹²
R¹⁵, R¹⁶ independently have the same meaning as R¹², R¹³;
R¹⁷ has the same meaning as R¹²;
R¹⁸ has the same meaning as R¹²
R¹⁹, R²⁰ independently have the same meaning as R¹², R¹³;
R²¹ has the same meaning as R¹²;
R²² has the same meaning as R¹²;
aryl is phenyl, naphth-1-yl or naphth-2-yl
heteroaryl is selected from the group consisting of 5- and 6- membered monocyclic and 9- or 10-membered bicyclic heterocycles containing one or more heteroatoms from the group consisting of N, O, and S;
m is 1, 2 or 3
or a pharmaceutically acceptable salt thereof.

3. A compound according to the general formula (I) 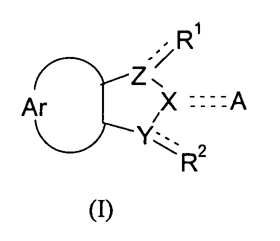 wherein the symbols Ar, A, X, Y, and Z and the substituents R¹ and R² have the meaning defined in claim 1 or 2, or a pharmaceutically acceptable salt thereof, for use as a pharmaceutical.

4. The use of a compound of formula (I) 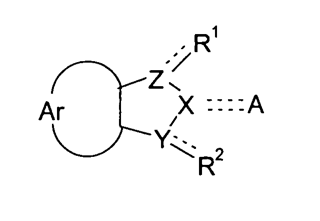 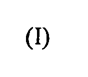 wherein the symbols Ar, A, X, Y, and Z and the substituents R¹ and R² have the meanings defined in claim 1 or 2, or a pharmaceutically acceptable salt thereof, for the manufacture of a pharmaceutical for the inhibition of DNMTs, more particularly DNMT1, and/or the inhibition of DNA methylation.

5. The use according to claim 4, wherein the pharmaceutical is for the treatment of a disease associated with aberrant DNA methylation.

6. The use according to claim 5, wherein the disease is a developmental disorder or a proliferative disesase.

7. The use according to claim 6, wherein the disease is Prader-Willi-Syndrome, Angelman-Syndrome (Happy Puppet Syndrome), Beckwith-Wiedemann-Syndrome, coronary restenosis, neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, prostate carcinoma, or plasmocytoma.

8. The use according to claim 7, wherein the disease is colon carcinoma, familiary adenomatous polyposis carcinoma and hereditary non-polyposis colorectal cancer, prostate carcinoma, melanoma, non-Hodgkin lymphoma, acute lymphatic leukemia (ALL), chronic lymphatic leukemia (CLL), acute myeolid leukemia (AML), chronic myeloid leukemia (CML), or hepatocellular carcinoma.

9. The use according to claim 7, wherein the disease is Prader-Willi-Syndrome, Angelman-Syndrome (Happy Puppet Syndrome), Beckwith-Wiedemann-Syndrome.

10. The use according to any of claims 1 to 9, wherein the pharmaceutical is co-administered with a compounds selected from the group consisting of (i) antimetabolites, cytarabine, fludarabine, 5-fluoro-2'-deoxyuridine, gemcitabine, hydroxyurea or methotrexate; (ii) DNA-fragmenting agents, bleomycin, (iii) DNA-crosslinking agents, chlorambucil, cisplatin, fotemustine, cyclophosphamide or nitrogen mustard; (iv) intercalating agents, adriamycin (doxorubicin) or mitoxantrone; (v) protein synthesis inhibitors, L-asparaginase, cycloheximide, puromycin or diphteria toxin; (vi) topoisomerase I poisons, camptothecin or topotecan; (vii) topoisomerase II poisons, etoposide (VP-16) or teniposide; (viii) microtubule-directed agents, colcemid, colchicine, paclitaxel (taxol), docetaxel (taxotere), vinblastine or vincristine; (ix) kinase inhibitors, flavopiridol, staurosporin, STI571 (CPG 57148B) or UCN-01 (7-hydroxystaurosporine); (x) miscellaneous investigational agents, trichostatin A, thioplatin, PS-341, phenylbutyrate, ET-18-OCH3, or farnesyl transferase inhibitors (L-739749, L-744832); polyphenols, quercetin, resveratrol, piceatannol, epigallocatechine gallate, theaflavins, flavanols, procyanidins, betulinic acid and derivatives thereof; (xi) hormones, glucocorticoids or fenretinide; (xii) hormone antagonists, tamoxifen, finasteride or LHRH antagonists, (xiii) demethylating agents, 5-azacytidine, 5-aza-2'deoxycytidine, 5,6-dihydro-5-azacytidine, or (xiv) a combination of any of the pharmaceuticals given above.

11. The use according to any of claims 4 to 10, wherein the pharmaceutical is for the induction of cellular differentiation.

12. The use according to any of claims 4 to 10, wherein the pharmaceutical is for the treatment of infections.

13. A pharmaceutical preparation comprising an effective dose of at least one compound of the formula (I) as defined in claim 1 or 2 and/or a pharmaceutically acceptable salt thereof and a pharmaceutically acceptable carrier.
